# EUROPEAN PATENT APPLICATION

(11) **EP 4 137 833 A1**
(43) Date of publication of application: **22.02.2023**
(21) Application number: 21198791.2
(22) Date of filing: 24.09.2021
(51) Int. Cl.: G01R 33/28, G01R 33/30

(54) **MATTRESS FOR AN EXAMINATION TABLE OF A MRI APPARATUS EQUIPPED WITH A CABLE DUCT**

(30) Priority: 20.08.2021 WO PCT/CN2021/113712
(71) Applicant: Koninklijke Philips N.V., 5656 AG Eindhoven (NL)
(72) Inventor: DENG, Wen (Wayne), Eindhoven (NL); HUA, Hao, Eindhoven (NL); BLOEMERS, Koert, Eindhoven (NL)
(74) Representative: Philips Intellectual Property & Standards

(57) **Abstract**

The invention relates to an arrangement (1) with a mattress (2) for an examination table (18) of a magnetic resonance apparatus (6) and a cable duct (3) which is arranged along a side of the mattress (2), wherein
the cable duct (3) comprises an outer wall (4) which at least partly surrounds a cable reception volume (5) in which cables of the magnetic resonance apparatus (6) may be arranged,
the outer wall (4) has a longitudinal slit (7) between two slit-confining outer wall areas (19), wherein the slit extends parallel to the side of the mattress (2) along at least part of the length of the cable duct (3), and
the longitudinal slit (7) is configured for allowing a cable to be inserted into or removed from the cable reception volume (5) via the longitudinal slit (7).

In this way, a possibility for efficient cable management which also promotes the comfort of the patient is provided.

## Description

### FIELD OF THE INVENTION

The invention relates to the field of mattresses, and in particular to mattresses for supporting patients under examination, especially in a magnetic resonance (MR) apparatus.

### BACKGROUND OF THE INVENTION

For MR examination, persons under examination are usually positioned on an examination table.

During a respective MR scan, there are usually many cables on the examination table such as coil cables, interface cables, headset cables and nurse call cables. On many coil and interface cables, there is an additional thermal part called balun which becomes hot during the MR scan. Managing the cables, especially those with balun, on such an examination table of a MR system is difficult and annoying, especially for the following problems. Due to the lack of proper cable management method, the coil cables with balun have to be placed on the body of the person who is under examination. This leads to a risk of balun burning of the person. To mitigate the risk of burning, typically, an additional spacer is required to be placed between the balun and the person. Further, the coil setup gets even more complicated when more coils are necessary for examination. In addition, there is a risk that the cables and baluns fall off from the examination table. Furthermore, the person's comfort is highly restricted due to the many items, including baluns, cables, and the spacer, which are arranged on the person's body.

From US 2013/176029 A1 a bed apparatus for a magnetic resonance imaging apparatus is known which includes a table, a table driving mechanism, a bed supporting part, and a cable guide. The table provides a connection port for a reception coil of magnetic resonance signals. The table driving mechanism is configured to shift the table. The bed supporting part is configured to support the table. The cable guide is configured to protect a signal cable between the table and the bed supporting part and to bend a portion of the signal cable along with a move of the table. The signal cable is connected to the connection port. The portion of the signal cable corresponds to a position of the table.

### SUMMARY OF THE INVENTION

It is an object of the invention to provide a possibility for efficient cable management which also promotes the comfort of the patient.

According to the invention, this object is addressed by the subject matter of the independent claims. Embodiments of the invention are described in the dependent claims.

Therefore, according to the invention, an arrangement with a mattress for an examination table of a magnetic resonance apparatus and a cable duct which is arranged along a side of the mattress is provided, wherein
the cable duct comprises an outer wall which at least partly surrounds a cable reception volume in which cables of the magnetic resonance apparatus may be arranged,
the outer wall has a longitudinal slit between two slit-confining outer wall areas, wherein the slit extends parallel to the side of the mattress along at least part of the length of the cable duct, and
the longitudinal slit is configured for allowing a cable to be inserted into or removed from the cable reception volume via the longitudinal slit.

In this way, an efficient cable management is provided which avoids annoying cables and baluns which could bother or even burn the patient.

In general, the cable duct could be arranged on any side of the mattress. The side of the mattress where the cable duct is arranged may be one of the two long sides of the mattress. Usually, this is the side from which a medical doctor or a nurse would prepare the patient for examination.

According to an embodiment of the invention, at least in a region of the outer wall which is adjacent to the longitudinal slit, the outer wall is made from a flexible material such that the outer wall is deformable for achieving an access opening via which a cable may be inserted into and/or a cable may be removed from the cable reception volume. In this way, the slit may be opened up sufficiently in order to insert or remove cables and baluns of different types and sizes. In this respect, according to an embodiment of the invention, in a natural state in which no access opening is formed by deforming the flexible material of the outer wall by an external force, the two slit-confining outer wall areas between which the longitudinal slit is formed are in contact with each other such that the cable reception volume is completely surrounded by the outer wall. This has the advantage that a cable or balun which has been inserted into the cable reception volume of the cable duct cannot be accessed and touched accidentally from outside of the cable duct. According to an alternative embodiment of the invention, in a natural state in which no access opening is formed by deforming the flexible material of the outer wall by an external force, the two slit-confining outer wall areas between which the longitudinal slit is formed are at a distance from each other such that the longitudinal slit forms a permanent longitudinal opening. According to this alternative, the cable duct may be arranged relative to the mattress in such a way that in the natural state the longitudinal opening of the outer wall faces a side portion of the mattress on a side of the mattress such that the cable reception volume is closed by the outer wall and the side portion of the mattress which is faced by the permanent longitudinal opening, and in a deformed state in which the access opening is formed by deforming the flexible material of the outer wall the cable reception volume is accessible via the access opening between of the outer wall and the side portion of the mattress which is faced by the longitudinal opening. In this way, a cable or balun which has been inserted into the cable reception volume of the cable duct cannot be accessed and touched accidentally from outside of the cable duct, either, since the cable reception volume is completely confined by the outer walls and the side of the mattress.

The invention allows for many different ways of combining the cable duct and the mattress. According to an embodiment of the invention, the cable duct is formed by a mattress accessory which comprises a middle section and a side section, wherein the middle section is arranged below the mattress and the side section protrudes over a side edge on a side of the mattress and forms the cable duct. While in this way, a cable duct could also be provided on just one side of the mattress, it is easy to provide a cable duct on two opposite sides of the mattress. Further, the mattress accessory with the cable duct(s) is held in place by the weight of the mattress and also by the weight of the patient in case of an examination situation. In this respect, according to an embodiment of the invention, the mattress accessory comprises two side sections which are arranged on opposite sides of the middle part and which protrude over the side edges on the long sides of the mattress and form a respective cable duct on both long sides of the mattress.

According to an alternative embodiment of the invention, the cable duct is formed by a mattress accessory which comprises a side section and a fastening section wherein the mattress accessory is attached to the mattress via the fastening section such that the side section protrudes over a side edge on a side of the mattress and forms the cable duct. The mattress accessory may be attached to the mattress by hook-and-loop fastener or push buttons. Two such mattress accessories may be arranged on both long sides of the mattress, respectively.

Further, it is another option of the invention, that the mattress and the cable duct together form an integral part. In this respect, a second cable duct may be arranged on the other longs side of the mattress and is also integral with the mattress.

In general, the shape of the outer wall of the cable duct may follow different designs. However, according to an embodiment of the invention, the outer wall of the cable duct which surrounds the cable reception volume is C-shaped in a cross section which is perpendicular to the long side direction of the mattress. Further, the outer wall of the cable duct may be made from a foam material, e.g. from EVA (ethylene vinyl acetate) foam which is wrapped with a PU (polyurethane) layer on the outside. As for conventional mattresses, also the mattress according to the present invention may be made from a foam material. The foam material of the cable duct may be more flexible than the foam material of the mattress. Further, according to an embodiment of the invention, the longitudinal slit of the outer wall extends along the complete length of the cable duct. Furthermore, according to an embodiment of the invention, the outer wall of the cable duct and/or the mattress is/are pierced, e.g. for getting access to alignment grooves for some dedicated coils of a patient table on which the mattress with the cable duct is used, in case the alignment grooves are covered by the cable duct.

The invention also relates to an examination table for supporting a person under examination in a magnetic resonance apparatus with an arrangement as described above. The examination table may comprise a handle for manipulating the examination table, wherein the handle comprises a gripping area for gripping the handle with a human hand, and wherein the gripping area comprises a bore for the reception of a cable. This helps further avoiding that such a cable annoys the patient or a nurse or medical doctor which prepares the patient for examination.

The invention also relates to a magnetic resonance system with an examination table as described above.

Further, the invention relates to a cable management assembly for a magnetic resonance apparatus, comprising a mattress accessory including a side section and a fastening section, wherein
the fastening section is configured to be arranged to a mattress to position the side portion relative to the mattress so as to form a cable duct, wherein
the cable duct comprises an outer wall which at least partially surrounds a cable reception volume in which cables of the magnetic resonance apparatus may be arranged,
the outer wall has a longitudinal slit between two slit-confining outer wall areas along at least part of the length of the cable duct, and
at least in a region of the outer wall which is adjacent to the longitudinal slit the outer wall is made from a flexible material such that the outer wall is deformable for achieving an access opening via which a cable may be inserted into and/or a cable may be removed from the cable reception volume.

According to an embodiment of the invention, the mattress accessory comprises a middle section next to the side section, wherein the middle section is configured for being arranged below the mattress and the side section is configured for protruding over a side edge on a side of the mattress and forms the cable duct. According to an alternative embodiment of the invention, the mattress accessory is configured for being attached to the mattress via the fastening section such that the side section which forms the cable duct protrudes over a side edge on a side of the mattress.

Finally, the invention also relates to a method of use of a cable duct for a mattress of a magnetic resonance apparatus, wherein
the cable duct comprises an outer wall which surrounds a cable reception volume in which cables of the MR apparatus may be arranged,
the outer wall has a longitudinal slit between two slit-confining outer wall areas which extends parallel to a side of the mattress along at least part of the length of the cable duct, and
at least in a region of the outer wall which is adjacent to the longitudinal slit the outer wall is made from a flexible material, such that the outer wall is deformable for achieving an access opening,
comprising the following method steps:
   arranging the cable duct along a side of the mattress,
   deforming the flexible material of the outer wall for achieving the access opening and keeping up the access opening,
   arranging at least one cable in the cable reception volume and/or removing at least one cable in the cable reception volume via the access opening,
   finishing the deforming of the flexible material of the outer wall.

### BRIEF DESCRIPTION OF THE DRAWINGS

These and other aspects of the invention will be apparent from and elucidated with reference to the embodiments described hereinafter. Such an embodiment does not necessarily represent the full scope of the invention, however, and reference is made therefore to the claims and herein for interpreting the scope of the invention.

In the drawings:
Fig. 1 schematically depicts a mattress accessory according to an embodiment of the invention in a perspective view,
Fig. 2 schematically depicts a section of the mattress accessory according to Fig. 1 together with a mattress in a perspective view,
Fig. 3 schematically depicts a detail of mattress accessory according to Fig. 1 together with a mattress in a perspective view,
Fig. 4a schematically depicts a detail of a mattress and a mattress accessory with an open slit in according to an embodiment of the invention a cross-sectional view,
Fig. 4b schematically depicts a detail of a mattress and a mattress accessory with a closed slit in a cross-sectional view,
Fig. 5 schematically depicts a detail of a MR apparatus with an examination table which is equipped with another mattress accessory according to an embodiment of the invention,
Fig. 6 schematically depicts a mattress together with two mattress accessories according to still another embodiment of the invention,
Fig. 7 schematically depicts a detail of a mattress and a mattress accessory which comprises an open slit on the top according to an embodiment of the invention in a cross-sectional view,
Fig. 8a schematically depicts a detail of an examination table with a handle according to an embodiment of the invention in a perspective view, and
Fig. 8b depicts the detail of the examination table of Fig. 8a wherein the depiction of the handle is omitted for better showing the routing of the cable.

### DETAILED DESCRIPTION OF EMBODIMENTS

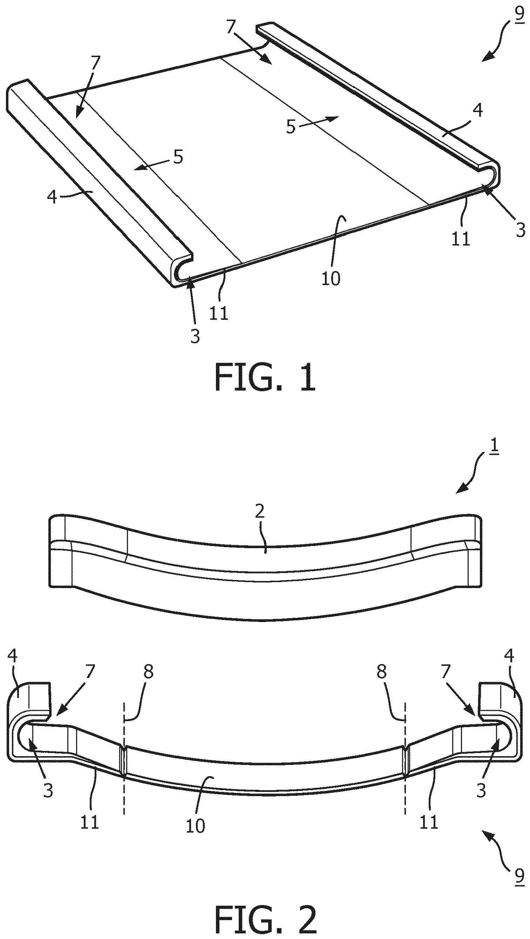

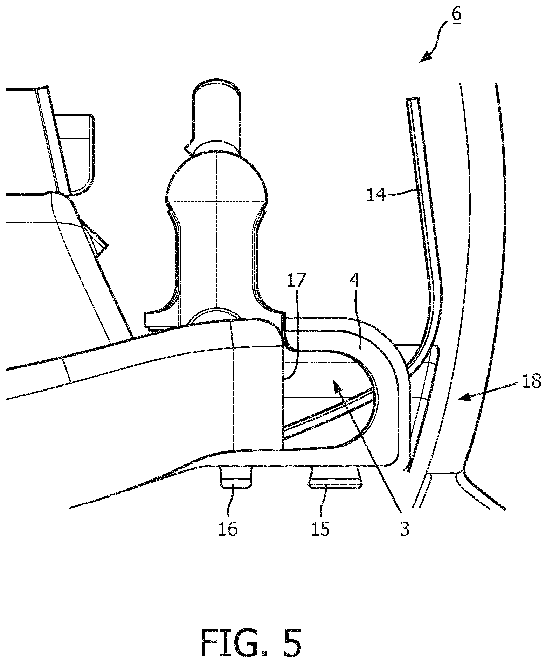

Fig. 1 schematically depicts a mattress accessory 9 according to an embodiment of the invention in a perspective view, and Fig. 2 schematically depicts a section of the mattress accessory 9 according to Fig. 1 together with a mattress 2 in a perspective view. In this way, an arrangement 1 with a mattress 2 and a cable duct 3 which is arranged along a side of the mattress 2 is provided, wherein the cable duct 3 runs parallel to an axis 8 which runs parallel to the long side of the mattress 2. This arrangement 1 is intended to be used for an examination table 18 of a magnetic resonance apparatus 6 a as shown in in Fig. 5, which schematically depicts a detail of such a magnetic resonance apparatus 6 with an examination table 18 which is equipped with a mattress accessory 9 according to an embodiment of the invention.

Reverting back to Figs. 1 and 2, the cable duct 3 comprises an outer wall 4 which partly surrounds a cable reception volume 5 in which cables of the magnetic resonance apparatus 6 may be arranged. The outer wall 4 has a longitudinal slit 7 between two slit-confining outer wall areas 19, wherein the slit 7 extends parallel to the side of the mattress 2 along the length of the cable duct 3, This longitudinal slit 7 is configured for allowing a cable to be inserted into or removed from the cable reception volume 5 via the longitudinal slit 7.

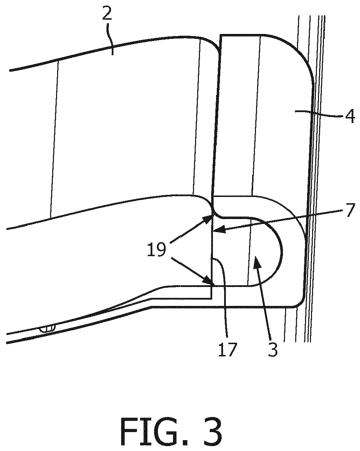

In a region of the outer wall 4 which is adjacent to the longitudinal slit 7 the outer wall 4 is made from a flexible material such that the outer wall 4 is deformable for achieving an access opening via which a cable may be inserted into and/or a cable may be removed from the cable reception volume 5. According to the embodiment shown in Figs. 1 and 2, in a natural state in which no access opening is formed by deforming the flexible material of the outer wall 4 by an external force, the two slit-confining outer wall areas 19 between which the longitudinal slit 7 is formed are at a distance from each other such that the longitudinal slit 7 forms a permanent longitudinal opening. In order to close this opening for avoiding that a cable which is inserted into the cable duct 3 may accidentally be touched, the cable duct 3 is arranged relative to the mattress 2 in such a way that in the natural state the longitudinal opening of the outer wall 4 faces a side portion 17 of the mattress 2 on a side of the mattress 2 such that the cable reception volume 5 is closed by the outer wall 4 and the side portion 17 of the mattress 2 which is faced by the permanent longitudinal opening, and in a deformed state in which the access opening is formed by deforming the flexible material of the outer wall 4 the cable reception volume 5 is accessible via the access opening between of the outer wall 4 and the side portion 17 of the mattress 2 which is faced by the longitudinal opening 7. Fig. 3 which schematically depicts a detail of the mattress accessory 9 according to Fig. 1 together with a mattress 2 in a perspective view shows the outer wall 4 in its natural undeformed state. Further, as depicted in Figs. 1, 2, and 3, the outer wall 4 of the cable duct 3 is C-shaped with its opening facing a side of the mattress 2.

As depicted in Fig. 2, the mattress accessory 9 comprises a middle section 10 and two side section 11, wherein the middle section 10 is arranged below the mattress 2 and the side sections 11 protrude over the side edges on both sides of the mattress 2. In this way, the mattress accessory 9 with the cable ducts 3 may be held in place by the weight of the mattress 2 and also by the weight of the patient in case of an examination situation.

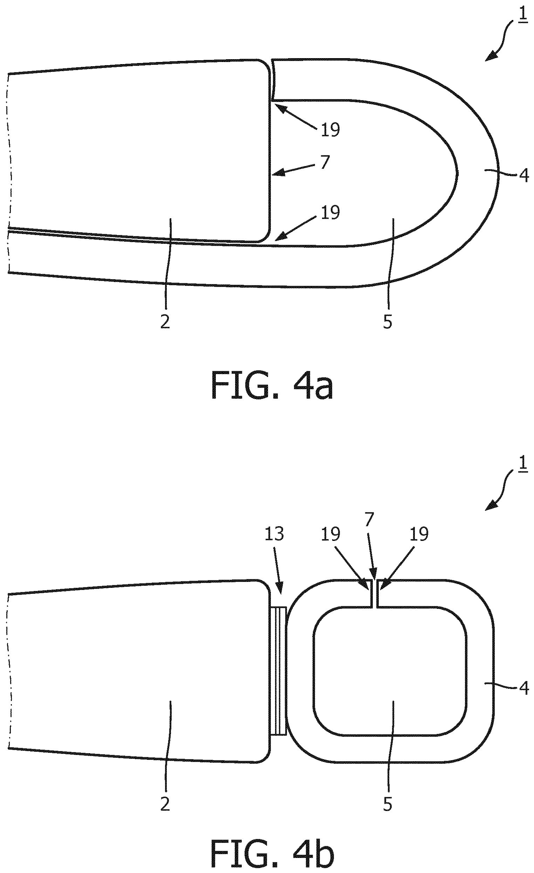

As mentioned above and as also depicted in Fig. 4a, according to an embodiment of the invention, the mattress accessory 9 is provided with an open slit 7, wherein the slit-confining outer walls 19 between which the slit 7 is formed are arranged in such a way relative to the side of the mattress 2 that the cable reception volume is circumferentially closed. As mentioned above, in this way, cables which are arranged in the cable reception volume 5 cannot be touched accidentally. According to an alternative design which is shown in Fig. 4b the slit 7 may also be closed in a natural state in which the flexible regions of the outer wall 4 of the cable duct 3 are not deformed by an externa force. Therefore, according to this embodiment, in the natural state in which no access opening is formed by deforming the flexible material of the outer wall 4 by an external force, the two slit-confining outer wall areas 19 between which the longitudinal slit 7 is formed are in contact with each other such that the cable reception volume 5 is completely surrounded by the outer wall 4. Hence, in case of this embodiment it is not necessary to use a part of the mattress 2 for closing the cable reception volume 5 circumferentially. Therefore, according to this embodiment of the invention, the cable duct 3 is formed by a mattress accessory 9 which comprises a side section 11 and a fastening section 12 wherein the mattress accessory 9 is attached to the mattress 2 via the fastening section 12 such that the side section 11 protrudes over a side edge on a side of the mattress 2 and forms the cable duct 3. Here, the mattress accessory 9 is attached to the mattress 2 by a hook-and-loop fastener 13. Alternatively, for attaching the mattress accessory 9 to the mattress 2 push buttons (not shown) could be used. Also in case of this embodiment of the invention, two such mattress accessories 9 are arranged on both long sides of the mattress 2, respectively, for providing a cable duct 3 on both opposite long sides of the mattress 2.

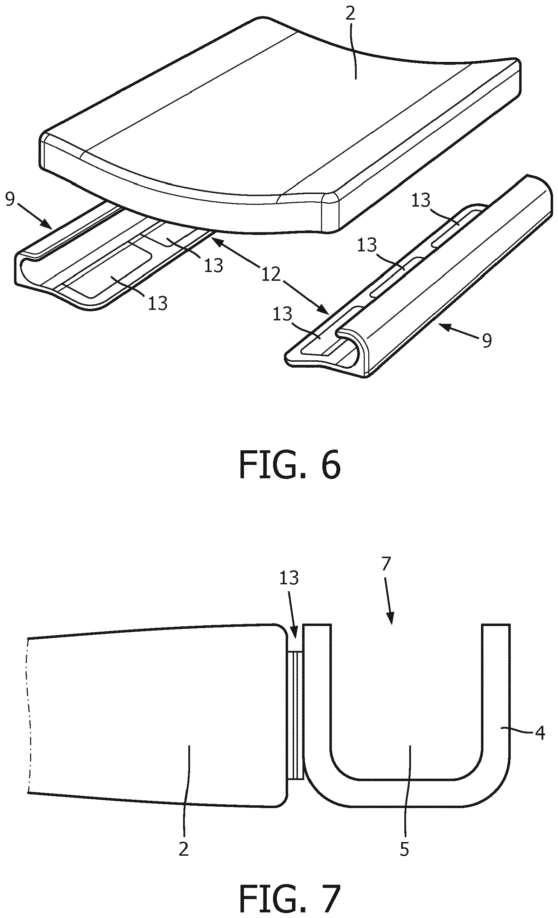

Fig. 6 schematically depicts a mattress together with two mattress accessories 9 according to still another embodiment of the invention. In similar way as for the embodiment of Fig. 4b, the mattress accessories 9 are attached to the two opposite long sides of the mattress 2, respectively. The mattress accessories 9 also comprise hook-and-loop fasteners 13 for attaching them to the mattress 2. However, in addition the mattress accessories 9 each comprise a region which extends under the mattress 2 and acts as a fastening section 12 such that the hook-and-loop fasteners 13 may interact with respective counter parts of the mattress 2 which are arranged on the bottom of the mattress 2. Similar to the embodiment shown in Figs. 1, 2, and 3, this allows for a design of the cable duct 3 with C-shaped outer walls 4 which provide for a permanent slit 7 which is closed by a side of the mattress 2 when the mattress accessories 9 are attached to the mattress 2.

As can be seen from Fig. 7 which schematically depicts a detail of a mattress 2 and a mattress accessory 9 which comprises an open slit 7 on the top, also in case of a permanently open slit 7 it is not absolutely necessary to cover the slit 7. When a cable is arranged in the cable reception volume 5 it will typically lie on the bottom of the reception volume 5 and, hence, it is less likely that the cable is accidentally touched compared with a situation in which the cable is placed on the mattress 2 next to the patient.

As already mentioned above, Fig. 5 schematically depicts a detail of a MR apparatus with an examination table. From Fig. 5 it may also be understood, that according to an embodiment of the invention the examination table may be equipped with an arm support 14 for the patient. Further, the examination table may comprise a strap opening 15 and a coil cable opening 16. In order to provide a possibility of reaching the strap opening 15 and the coil cable opening 16 of the examination table 18 in case these are covered by the cable duct 3 the flexible outer wall 4 can be folded up to access these strap opening 15 and the coil cable opening 16.

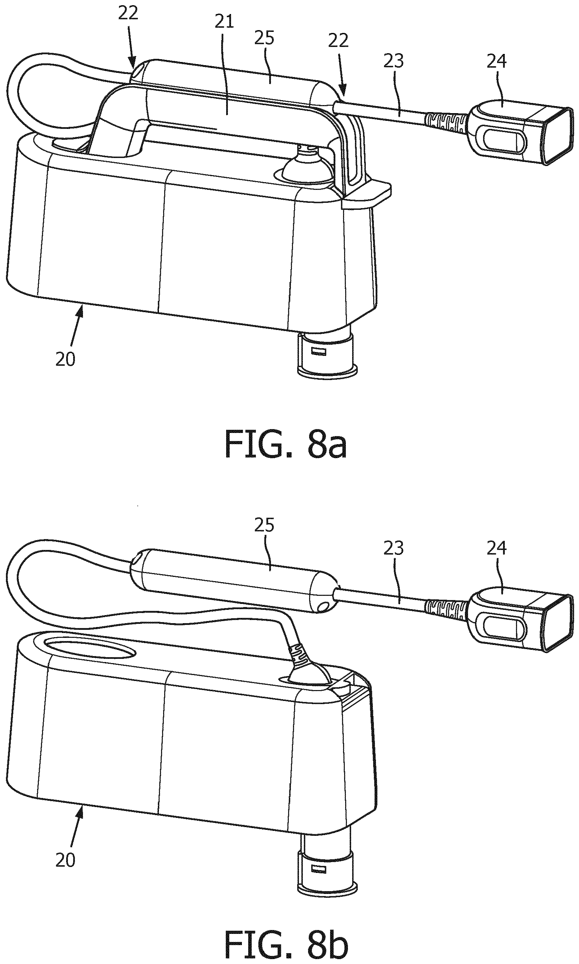

Finally, Fig. 8a schematically depicts an adaptor interface 20 of an examination table with a handle 21 according to an embodiment of the invention in a perspective view. The adaptor interface 20 comprises a cable 23 with a balun 25 and a connector 24 which provides a connectivity for a coil cable (not shown). The handle 21 provides a gripping area and comprises a groove 22 to accommodate the cable 23 and/or the balun 25 of the cable 23. This helps avoiding that the cable 23 and/or the balun 25 annoys the patient or a nurse or medical doctor which prepares the patient for examination. Fig. 8b, in general depicts the same adaptor interface 20 as Fig. 8a wherein the depiction of the handle 21 is omitted for better showing the routing of the cable 23.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Further, for the sake of clearness, not all elements in the drawings may have been supplied with reference signs.

**REFERENCE SYMBOL LIST**

| | |
|---|---|
| arrangement | 1 |
| mattress | 2 |
| cable duct | 3 |
| outer wall | 4 |
| cable reception volume | 5 |
| MR apparatus | 6 |
| longitudinal slit | 7 |
| axis which runs parallel to the long side of the mattress | 8 |
| mattress accessory | 9 |
| middle section | 10 |
| side section | 11 |
| fastening section | 12 |
| hook-and-loop fastener | 13 |
| arm support | 14 |
| strap opening | 15 |
| coil cable opening | 16 |
| side portion on the long side of the mattress | 17 |
| examination table | 18 |
| slit-confining outer wall areas | 19 |
| adaptor interface | 20 |
| handle | 21 |
| groove | 22 |
| cable | 23 |
| connector | 24 |
| balun | 25 |

## Claims

1. Arrangement (1) with a mattress (2) for an examination table (18) of a magnetic resonance apparatus (6) and a cable duct (3) which is arranged along a side of the mattress (2), wherein
the cable duct (3) comprises an outer wall (4) which at least partly surrounds a cable reception volume (5) in which cables of the magnetic resonance apparatus (6) may be arranged,
the outer wall (4) has a longitudinal slit (7) between two slit-confining outer wall areas (19), wherein the slit extends parallel to the side of the mattress (2) along at least part of the length of the cable duct (3), and
the longitudinal slit (7) is configured for allowing a cable to be inserted into or removed from the cable reception volume (5) via the longitudinal slit (7).

2. Arrangement (1) according to claim 1, wherein at least in a region of the outer wall (4) which is adjacent to the longitudinal slit (7) the outer wall (4) is made from a flexible material such that the outer wall (4) is deformable for achieving an access opening via which a cable may be inserted into and/or a cable may be removed from the cable reception volume (5).

3. Arrangement (1) according to claim 2, wherein in a natural state in which no access opening is formed by deforming the flexible material of the outer wall (4) by an external force, the two slit-confining outer wall areas (19) between which the longitudinal slit (7) is formed are in contact with each other such that the cable reception volume (5) is completely surrounded by the outer wall (4).

4. Arrangement (1) according to claim 2, wherein in a natural state in which no access opening is formed by deforming the flexible material of the outer wall (4) by an external force, the two slit-confining outer wall areas (19) between which the longitudinal slit (7) is formed are at a distance from each other such that the longitudinal slit (7) forms a permanent longitudinal opening.

5. Arrangement (1) according to claim 4, wherein the cable duct (3) is arranged relative to the mattress (2) in such a way that in the natural state the longitudinal opening of the outer wall (4) faces a side portion (17) of the mattress (2) on a side of the mattress (2) such that the cable reception volume (5) is closed by the outer wall (4) and the side portion (17) of the mattress (2) which is faced by the permanent longitudinal opening, and
in a deformed state in which the access opening is formed by deforming the flexible material of the outer wall (4) the cable reception volume (5) is accessible via the access opening between of the outer wall (4) and the side portion (17) of the mattress (2) which is faced by the longitudinal opening (7).

6. Arrangement (1) according to any of the previous claims, wherein the cable duct (3) is formed by a mattress accessory (9) which comprises a middle section (10) and a side section (11), wherein the middle section (10) is arranged below the mattress (2) and the side section (11) protrudes over a side edge on a side of the mattress (2) and forms the cable duct (3).

7. Arrangement (1) according to claim 6, wherein the mattress accessory (9) comprises two side sections (11) which are arranged on opposite sides of the middle part (10) and which protrude over the side edges on the long sides of the mattress (2) and form a respective cable duct (3) on both long sides of the mattress (2).

8. Arrangement (1) according to any of claims 1 to 5, wherein the cable duct (3) is formed by a mattress accessory (9) which comprises a side section (11) and a fastening section (12) wherein the mattress accessory (9) is attached to the mattress (2) via the fastening section (12) such that the side section (11) protrudes over a side edge on a side of the mattress (2) and forms the cable duct (3).

9. Arrangement (1) according to any of claims 1 to 7, wherein the mattress (2) and the cable duct (3) together form an integral part.

10. Magnetic resonance system (6) with arrangement (1) according to claim any of claims 1-9.

11. Cable management assembly for a magnetic resonance apparatus (6), comprising a mattress accessory (9) and/or an adaptor interface (20), wherein
the mattress accessory (9) comprises a side section (11) and a fastening section (12), wherein the fastening section (12) is configured to be arranged to a mattress (2) to position the side portion relative to the mattress (3) so as to form a cable duct (3),
the cable duct (3) comprises an outer wall (4) which at least partially surrounds a cable reception volume (5) in which cables of the magnetic resonance apparatus (6) may be arranged,
the outer wall (4) has a longitudinal slit (7) between two slit-confining outer wall areas (19) along at least part of the length of the cable duct (3), and
at least in a region of the outer wall (4) which is adjacent to the longitudinal slit (7) the outer wall (4) is made from a flexible material such that the outer wall (4) is deformable for achieving an access opening via which a cable may be inserted into and/or a cable may be removed from the cable reception volume (5); and
the adaptor interface (20) comprises a handle (21) and a cable (23), wherein the cable (23) comprises a connector (24) which provides a connectivity for a coil cable, and the handle (21) provides a gripping area and comprises a groove (22) to accommodate the cable (23) and/or a balun (25) of the cable (23).

12. Cable management assembly according to claim 11, wherein the mattress accessory (9) comprises a middle section (10) next to the side section (11), wherein the middle section (10) is configured for being arranged below the mattress (2) and the side section (11) is configured for protruding over a side edge on a side of the mattress (2) and forms the cable duct (3).

13. Cable management assembly according to claim 11, wherein the mattress accessory (9) is configured for being attached to the mattress (2) via the fastening section (12) such that the side section (11) which forms the cable duct (3) protrudes over a side edge on a side of the mattress (2).

14. Method of use of a cable duct (3) for a mattress (2) of a magnetic resonance apparatus (6), wherein
the cable duct (3) comprises an outer wall (4) which surrounds a cable reception volume (5) in which cables of the MR apparatus (6) may be arranged,
the outer wall (4) has a longitudinal slit (7) between two slit-confining outer wall areas (19) which extends parallel to a side of the mattress (2) along at least part of the length of the cable duct (3), and
at least in a region of the outer wall (4) which is adjacent to the longitudinal slit (7) the outer wall (4) is made from a flexible material, such that the outer wall (4) is deformable for achieving an access opening,
comprising the following method steps:
arranging the cable duct (3) along a side of the mattress (2),
deforming the flexible material of the outer wall (4) for achieving the access opening and keeping up the access opening,
arranging at least one cable in the cable reception volume (5) and/or removing at least one cable in the cable reception volume (5) via the access opening,
finishing the deforming of the flexible material of the outer wall (4).
